# FASCICULE DE BREVET EUROPEEN

(11) **EP 1 570 808 B1**
(45) Date de publication et mention de la délivrance du brevet: **25.03.2009**
(21) Numéro de dépôt: 05290397.8
(22) Date de dépôt: 22.02.2005
(51) Int. Cl.: A61F 2/06

(54) **Endoprothèse à marqueurs pour conduit d'un corps vivant**
Stent mit Markierungen für eine Röhre im lebenden Körper
Stent with markers for a living body conduit

(30) Priorité: 03.03.2004 FR 0402215
(43) Date de publication de la demande: 07.09.2005
(73) Titulaire: B. Braun Medical SAS, 92100 Boulogne Billancourt (FR)
(72) Inventeur: Blanloeil, Patrick, 86240 Fontaine-le-Comte (FR); Forber, Simon, 86000 Poitiers (FR); Charret, Nathalie, Rés. Le Clos des Coudres, 86441 Migne-Auxences (FR)
(74) Mandataire: Schmit, Christian Norbert Marie

(56) Documents cités:
- EP-A- 1 362 564
- WO-A-03/061502
- US-A1- 2002 103 528
- US-A1- 2002 143 386
- US-A1- 2004 015 228
- US-B1- 6 334 871

## Description

La présente invention concerne une endoprothèse destinée à être implantée dans un conduit d'un corps vivant, munie de marqueurs radio-opaques, et un procédé de pose de marqueurs sur de telles endoprothèses.

On connaît de nombreuses endoprothèses d'angioplastie, destinées à être implantées dans un conduit du corps humain. Le positionnement de ces endoprothèses lors de leur pose est en général assuré par des marqueurs radio-opaques fixés à l'extrémité du cathéter d'introduction, ou à l'endoprothèse elle-même, qui peuvent être détectés avec des appareils radiographiques. Cette dernière solution est avantageuse parce qu'elle permet de situer l'endoprothèse dans le corps humain après retrait du cathéter, et permet ainsi d'effectuer d'autres interventions sur le conduit endommagé, par exemple le retrait de l'endoprothèse, l'ajout d'une seconde endoprothèse, ou toute autre opération dans laquelle la connaissance du positionnement de l'endoprothèse est utile.

On connaît déjà de nombreuses endoprothèses munies de tels marqueurs, ces derniers étant généralement constitués d'une matière métallique. Ces endoprothèses sont généralement en métal et expansibles car elles doivent être déformées lors de la pose et garder une forme tubulaire pour ménager un passage dans le conduit qui doit être renforcé après la pose.

On connaît de nombreux procédés destinés à rendre radio-opaques certaines parties de la prothèse. Par exemple le brevet EP-0679372 décrit des endoprothèses revêtues sur une partie de leur surface d'un matériau radio-opaque. Ce type de marquage est cependant peu satisfaisant car l'épaisseur de matériau est très faible, et le marquage est donc peu visible.

L'invention concerne des endoprothèses munies de véritables marqueurs radio-opaques, c'est-à-dire telles qu'en certains endroits d'une armature tubulaire se trouvent des éléments marqueurs en matériau radio-opaque situés sur une grande partie au moins de l'épaisseur de l'armature afin de pouvoir être facilement détectés.

On connaît de nombreux procédés pour fixer de tels marqueurs, en général par des moyens mécaniques ou par soudage. Ainsi, le brevet US-6 409 752 décrit des endoprothèses munies de marqueurs radio-opaques électro-soudés dans des trous de l'armature qui débouchent aux parois interne et externe d'une armature tubulaire. De même, le brevet US-6 471 721 décrit des endoprothèses munies de marqueurs radio-opaques électro-soudés ou soudés par diffusion dans une cavité d'une armature à orifices. Le résultat obtenu est peu satisfaisant. En effet, le procédé d'électro-soudure ou de soudure par diffusion est difficilement contrôlable et reproductible. En outre, il existe après la pose une corrosion galvanique des deux métaux au contact d'un liquide corporel ionisé, tel que le sang.

D'autres procédés de tenue mécanique d'un marqueur dans l'épaisseur d'une endoprothèse, tel que le sertissage décrit dans la demande internationale WO 02/078 762, ou le rivetage décrit dans le brevet US-6 402 777, sont difficilement contrôlables, donc reproductibles, et posent les mêmes problèmes de corrosion à l'utilisation. En outre, les déformations plastiques subies par le métal créent des amorces de corrosion.

Tous les procédés précités ont l'inconvénient de nécessiter, après la fixation des marqueurs, d'autres opérations de finition, comprenant au moins un polissage et une passivation.

Un autre procédé connu comprend, à la place du soudage ou du sertissage du marqueur, son collage dans une cavité de dimension et forme lui correspondant presque parfaitement ; le marqueur et les parois du trou sont enduits de colle, puis mis en contact. Selon ce procédé, le métal de l'armature et celui du marqueur sont au contact du liquide corporel ionisé qui circule, si bien qu'il existe un couple électrochimique qui provoque une corrosion localisée, sauf si les métaux sont choisis afin qu'ils aient le même potentiel électrochimique. Cette condition limite extrêmement les couples de métaux qui peuvent être utilisés.

On connaît aussi d'autres procédés de fixation de matériau radio-opaque sur une grande partie de l'épaisseur de l'armature d'une endoprothèse. Par exemple, on sait couler un mélange de particules radio-opaques et de matière durcissable dans une cavité de l'armature. Ce procédé, décrit dans la demande de brevet EP-1 216 665, évite les problèmes précités de tenue et de corrosion. Cependant, comme une partie de l'épaisseur du matériau est formée de la matière durcissable qui n'est pas radio-opaque, le marqueur obtenu a une faible opacité.

Selon une variante du procédé décrit dans ce document EP-1 216 665, un marqueur est simplement collé dans une cavité formée dans une armature. Le marqueur est plus petit que la cavité et a une position variable dans celle-ci. Comme la cavité ne débouche pas, le marqueur n'occupe qu'une partie de l'épaisseur de l'armature et a donc une opacité réduite. Les marqueurs décrits dans ce document ont cependant l'avantage d'isoler totalement le métal du marqueur du liquide corporel ionisé qui circule, tel que le sang, et donc de supprimer la corrosion due au couple électrochimique.

L'invention a pour objet une endoprothèse présentant les avantages des endoprothèses qui ne créent pas un couple électrochimique, c'est-à-dire dont le métal du marqueur est isolé du liquide corporel qui circule.

L'invention a aussi pour objet une endoprothèse présentant les avantages des endoprothèses dont le positionnement par rapport à l'armature est précis, c'est-à-dire dont le marqueur est au contact direct de l'armature.

L'invention a aussi pour objet une endoprothèse présentant les avantages des endoprothèses dont le marqueur est au contact direct de l'armature, à un même potentiel électrique, c'est-à-dire qu'aucun champ électrique n'est créé à proximité du marqueur par une différence de potentiel entre le marqueur et l'armature.

L'invention a aussi pour objet une endoprothèse présentant les avantages des endoprothèses qui ne nécessitent aucune opération après la pose des marqueurs, telle que des opérations de finition, notamment un polissage et une passivation.

Plus précisément, l'invention concerne une endoprothèse destinée à être implantée dans un conduit d'un corps vivant comprenant une armature sensiblement tubulaire comprise entre deux extrémités longitudinales et qui a une surface interne et une surface externe, l'armature ayant au moins un trou débouchant par deux orifices qui diffèrent au moins par la forme ou la dimension, et situés respectivement sur la surface interne et sur la surface externe de l'armature tubulaire, et un marqueur radio-opaque ayant au moins une forme ou une dimension telle que, avec son orientation dans le trou, il peut passer entièrement par l'un des deux orifices mais pas par l'autre, le marqueur étant logé au moins presque totalement dans le trou. Selon l'invention, le marqueur comprend une partie en contact avec au moins plusieurs emplacements distants de la paroi du trou qui sont plus proches d'un orifice du trou que de l'autre, et un matériau adhésif qui remplit la partie de trou qui n'est pas occupée par le marqueur, jusqu'aux deux orifices.

De préférence, l'une au moins des surfaces interne et externe de l'armature, au niveau d'un orifice du trou, a une courbure, et le matériau adhésif est disposé avec une courbure correspondante.

Dans un mode de réalisation, l'armature se prolonge par au moins un oeillet relié à une des extrémités longitudinales de l'armature, et le trou est situé dans l'oeillet.

De préférence, toute la surface du marqueur qui n'est pas en contact avec les parois du trou est recouverte de matière adhésive.

Dans un mode de réalisation, le trou comporte une partie tronconique sur laquelle se trouvent les emplacements de contact avec le marqueur.

De préférence, le marqueur comporte une partie de forme cylindrique dont une circonférence comprend les emplacements de contact avec la paroi.

De préférence, la partie du marqueur qui est au contact de la paroi du trou bouche pratiquement la section du trou, si bien que la colle déposée par un des orifices ne peut pas atteindre l'autre orifice.

De préférence, la matière adhésive est choisie parmi les résines époxydes, polyuréthannes et polyesters.

L'invention concerne aussi un procédé de pose d'un marqueur dans un trou débouchant d'une endoprothèse, le trou ayant deux orifices qui diffèrent au moins par la forme ou la dimension, le marqueur ayant au moins une forme ou une dimension telle que, avec son orientation dans le trou, il peut passer entièrement par l'un des deux orifices mais pas par l'autre ; selon l'invention, le procédé comprend : l'introduction du marqueur dans le trou par l'orifice le plus grand jusqu'à ce qu'il soit au contact de la paroi du trou avant l'autre orifice, l'introduction de matériau adhésif fluide d'un premier côté du marqueur, le durcissement du matériau adhésif, l'introduction de matériau adhésif fluide de l'autre côté du marqueur, et le durcissement du matériau adhésif.

Dans un mode d'exécution, le procédé comprend, pendant l'introduction du matériau adhésif fluide du premier côté du marqueur, l'obturation de l'orifice placé de l'autre côté du marqueur.

De préférence, le matériau adhésif utilisé des deux côtés du marqueur est le même.

Dans un mode d'exécution avantageux, un matériau adhésif est de type polymérisable, et une étape de durcissement au moins de matériau adhésif est une polymérisation. Il est alors avantageux que l'étape de durcissement par polymérisation soit une photopolymérisation.

De préférence, le procédé comprend une étape de recuit postérieure à la polymérisation.

D'autres caractéristiques et avantages de l'invention seront mieux compris à la lecture de la description qui va suivre, faite en référence aux dessins annexés dans lesquels :
la figure 1 est une vue développée d'une extrémité d'un exemple d'endoprothèse ;
la figure 2 est une coupe de l'endoprothèse de la figure 1, à son état normal tubulaire, coupée par un plan passant au milieu d'oeillets représentés sur la figure 1 ;
la figure 3 est une vue analogue à la figure 2, mais correspond à une endoprothèse ayant un plus grand nombre d'oeillets ; et
les figures 4 à 7 sont des coupes illustrant la mise en oeuvre d'un procédé selon l'invention.

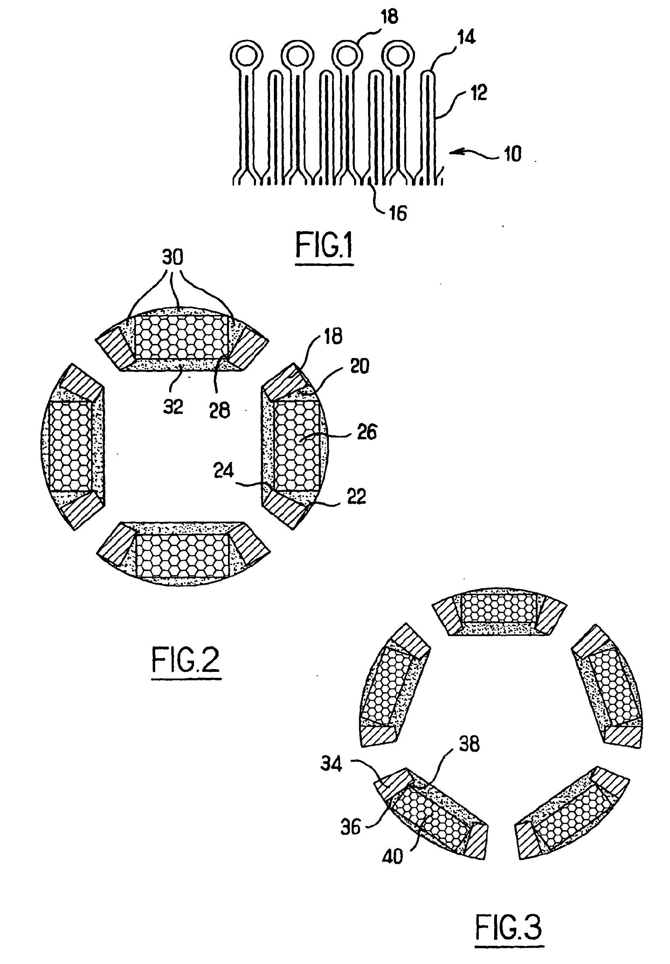

La figure 1 représente, sous forme développée dans un plan, l'extrémité d'une endoprothèse d'un type formé par découpe laser d'une mince feuille métallique, par exemple, de "Nitinol". L'endoprothèse peut s'élargir, à partir de l'état représenté sur la figure 1, lorsque les bras 12, articulés à des coudes 14 et ramifications 16, s'écartent les uns des autres. On note que certains bras se terminent à l'extrémité de l'endoprothèse par un oeillet 18. Celui-ci est aussi formé par découpe de la feuille métallique.

Les oeillets 18 sont destinés à contenir des marqueurs radio-opaques. Le rôle des marqueurs radio-opaques est de permettre à un chirurgien ou radiologue interventionniste de déterminer, par fluoroscopie, à quel endroit se trouve l'endoprothèse à l'intérieur d'un corps humain.

La figure 2 représente plus en détail les oeillets contenant des marqueurs d'une endoprothèse selon l'invention.

Sur la figure 2, qui représente en coupe quatre oeillets d'une même endoprothèse, en position comprimée, on note que chaque oeillet a un trou 20 de paroi tronconique. Cette paroi est comprise entre un orifice extérieur 22 et un orifice intérieur 24. La distance comprise entre ces deux orifices est de l'ordre du dixième de millimètre.

Un marqueur radio-opaque 26, par exemple formé de tantale, constitué d'un cylindre de section circulaire à extrémités plates dans cet exemple, est disposé dans le trou de manière que sa partie la plus à l'intérieur soit au contact de la paroi tronconique du trou 20, en un emplacement ou région 28 de contact (il s'agit en fait d'une circonférence de contact) qui est à une certaine distance de l'orifice interne 24. Ce contact peut être pratiquement linéaire, ou il peut être étendu, lorsque l'un au moins des deux matériaux de l'oeillet ou du marqueur se déforme à l'emplacement de contact.

Selon l'invention, si l'on suppose que l'oeillet 18 et le marqueur 26 sont circulaires, le diamètre du marqueur 26 est inférieur à celui de l'orifice extérieur 22 de l'oeillet mais supérieur au diamètre de l'orifice intérieur 24. De cette manière, par contact d'une de ses extrémités, le marqueur 26 est positionné par rapport à l'oeillet 18.

La référence 30 désigne une résine polymérisée qui fixe le marqueur 26 à l'oeillet 18 du côté extérieur. La référence 32 désigne aussi une résine polymérisée mais placée à l'intérieur.

On note que la résine 30, 32 isole totalement le marqueur 26 du courant de liquide corporel qui peut s'écouler à l'intérieur ou à l'extérieur de l'endoprothèse.

On note aussi que, grâce au contact entre le marqueur 26 et l'oeillet 18, ces deux éléments sont au même potentiel électrique et ne risquent pas de créer un champ électrique dans les parties qui leur sont proches.

Enfin, on note sur la figure 2 que la résine a donné une forme parfaite à l'endoprothèse, si bien que celle-ci ne nécessite aucune opération de finition.

La figure 3 représente une coupe, analogue à celle de la figure 2, d'une endoprothèse ayant à chaque extrémité cinq marqueurs et non quatre comme dans le cas de la figure 2. Dans cet exemple, on note que la surface conique interne du trou 36 de l'oeillet 34 forme un angle plus petit que dans le cas de l'endoprothèse de la figure 2. Par ailleurs, la surface de contact 38 d'un marqueur 40 avec cette surface interne 36 est relativement éloignée de l'orifice interne de l'oeillet. Cependant, les autres caractéristiques de cette endoprothèse sont analogues à celles de l'endoprothèse de la figure 2.

On décrit maintenant un exemple d'exécution du procédé de pose de marqueur sur une endoprothèse, selon l'invention.

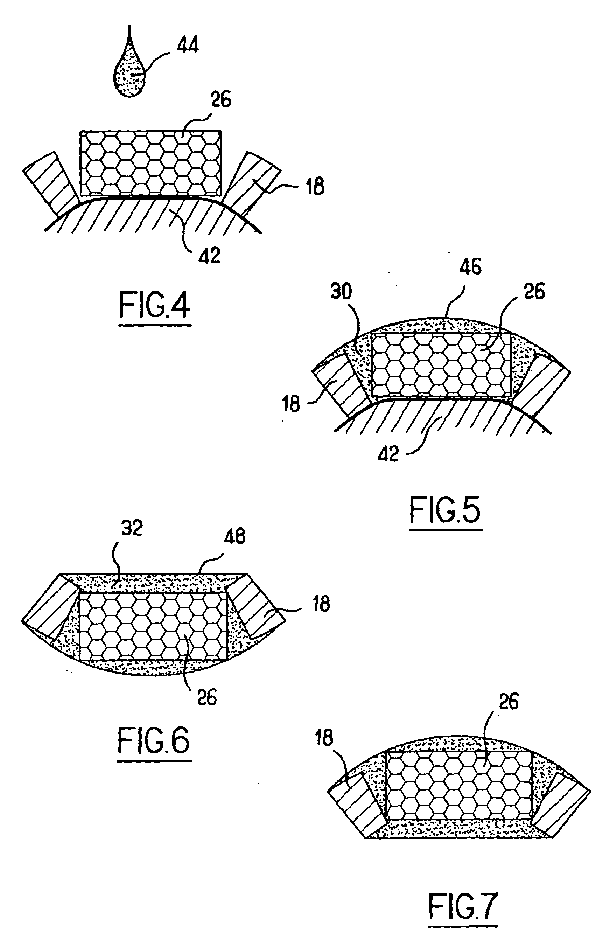

La figure 4 représente en coupe l'oeillet 18 dans lequel est placé un marqueur 26. On a représenté un mandrin 42 de matériau tendre, par exemple d'un élastomère de silicone, destiné à occuper une partie au moins de l'espace qui se trouve sous le marqueur 26 de manière que, lors de l'introduction d'une résine, celle-ci ne puisse pas passer indûment ou excessivement du côté inférieur sur la figure.

Une fois l'oeillet et le marqueur positionnés sur le mandrin 42, de la résine 44 est placée à la surface extérieure. La quantité de résine 44 est déterminée avec précision de manière que, comme indiqué sur la figure 5, elle forme une surface arrondie 46 tout autour de l'oeillet 18 et du marqueur 26.

En théorie, le marqueur 26 est au contact de l'oeillet 18 sur une circonférence complète et empêche le passage de résine du côté inférieur sur la figure. Cependant, étant donné la très petite dimension de ces éléments, il n'est pas toujours possible d'obtenir un ajustement parfait, et le mandrin 42 empêche l'écoulement excessif de résine à la partie inférieure.

Lorsque l'état représenté sur la figure 5 a été atteint, la résine 30 est soumise à une polymérisation, de préférence une photopolymérisation, qui assure ainsi la fixation du marqueur 26 dans l'oeillet 18.

Lorsque cette polymérisation a été suffisamment effectuée, l'endoprothèse est séparée du mandrin 42, et l'oeillet correspondant subit une rotation de 180° afin que la partie qui était inférieure se trouve à la partie supérieure. A ce moment, une goutte soigneusement mesurée de résine est appliquée et forme la résine 32 qui donne encore une surface très nette, grâce au réglage précis de la quantité mesurée, comme indiqué sur la figure 6. La résine 32 subit alors une polymérisation, de préférence une photopolymérisation.

Il est avantageux que l'ensemble de l'endoprothèse subisse ensuite un recuit destiné à assurer l'homogénéité de la polymérisation de l'ensemble de la résine. On obtient alors la disposition de la figure 7, qui correspond à un oeillet de la figure 2.

Il est avantageux d'utiliser comme matériau adhésif une résine contenant un inducteur de polymérisation radicalaire ; les réactions de polymérisation et de photopolymérisation de diverses résines, par exemple époxydes, acryliques, etc. étant bien connues des hommes du métier, elles ne sont donc pas décrites dans le présent mémoire. Il est préférable, mais non indispensable, que le matériau adhésif soit le même des deux côtés du marqueur.

On note sur la figure 7 que le marqueur obtenu présente les avantages d'opacité, de positionnement et de conduction électrique des marqueurs soudés ou sertis, l'épaisseur du marqueur étant élevée et donnant une opacité élevée. Il présente aussi les avantages des marqueurs qui sont enrobés dans une résine de sorte qu'il n'a pas de partie directement au contact d'un liquide corporel qui circule. Il ne possède pas les inconvénients de ces deux types de marqueurs, c'est-à-dire qu'il ne présente pas de corrosion due à un couple électrochimique, et il ne présente pas non plus l'inconvénient de nécessiter des étapes ultérieures de finition.

L'une des caractéristiques importantes du procédé de l'invention selon lequel les deux surfaces externe et interne de l'oeillet contenant le marqueur sont formées dans deux opérations différentes est qu'il permet l'obtention de surfaces parfaites des deux côtés. Cette caractéristique est très importante puisque, en cas de défaut d'aspect, c'est l'ensemble de l'endoprothèse qui doit être mis au rebut. Il est donc primordial d'obtenir un résultat aussi parfait que possible. Ce résultat est obtenu grâce au positionnement par contact du marqueur contre l'oeillet, et à la formation séparée des surfaces des deux côtés du marqueur.

Bien qu'on ait décrit un mode de réalisation particulier de l'invention, celle-ci n'est pas limitée aux éléments décrits. Ainsi, on a considéré que l'oeillet avait une forme de révolution et était destiné à loger un marqueur de forme cylindrique de section circulaire. Cette forme circulaire n'est pas indispensable. Par exemple, il est possible d'utiliser une forme ovale. En outre, il est aussi possible d'utiliser des formes qui ne sont pas parfaitement complémentaires, par exemple une forme de marqueur rectangulaire à coins arrondis dans un oeillet circulaire, dans la mesure où, lors de la fabrication, le mandrin qui ferme l'orifice inférieur empêche l'écoulement d'une quantité excessive de résine. D'autres sections que les formes circulaires, ovales ou rectangulaires précitées peuvent être utilisées pour le marqueur. En outre le marqueur n'est pas obligatoirement cylindrique, car il peut avoir une forme tronconique ou analogue dont la conicité est avantageusement inférieure à celle du trou de l'oeillet.

Bien qu'on ait décrit un trou formé dans un oeillet placé à l'extrémité de l'endoprothèse, il peut se trouver à tout emplacement choisi sur le corps de l'endoprothèse.

Une caractéristique essentielle est que, compte tenu de sa section, le marqueur, lorsqu'il est introduit dans l'oeillet avec une orientation convenable, est en contact en plusieurs emplacements avec l'oeillet. Une fois ce contact établi, il est facile de mettre en place la résine pour la fixation du marqueur.

On a représenté des marqueurs totalement entourés de résine vers l'extérieur et l'intérieur de l'endoprothèse. Cependant, si le métal de l'oeillet 18 et celui du marqueur 26 ont des potentiels électrochimiques très voisins et ne risquent donc pas de créer un couple électrochimique provoquant une corrosion, il est possible que le marqueur apparaisse à une surface. Par exemple, il peut former une partie de surface arrondie à la forme extérieure de l'endoprothèse.

Bien entendu, d'autres formes plus ou moins complexes peuvent être réalisées dans le cadre de l'invention, mais il faut se rappeler que, étant donné la très petite dimension des marqueurs et des oeillets, la véritable possibilité de variation mécanique est relativement limitée.

## Revendications

1. Endoprothèse destinée à être implantée dans un conduit d'un corps vivant comprenant :
une armature sensiblement tubulaire comprise entre deux extrémités longitudinales et qui a une surface interne et une surface externe,
l'armature ayant au moins un trou (20 ; 36) débouchant par deux orifices (22, 24) qui diffèrent au moins par la forme ou la dimension, et sont situés respectivement sur la surface interne et sur la surface externe de l'armature tubulaire, et
un marqueur radio-opaque (26 ; 40) ayant au moins une forme ou une dimension telle que, avec son orientation dans le trou, il peut passer entièrement par l'un des deux orifices (22) mais pas par l'autre (24), le marqueur étant logé au moins presque totalement dans le trou,
**caractérisée en ce que** le marqueur (26 ; 40) comprend une partie d'extrémité en contact avec au moins plusieurs emplacements (28 ; 38) de la paroi du trou (20 ; 36) qui sont plus proches d'un orifice (24) du trou que de l'autre (22), et
un matériau adhésif (30, 32) remplit la partie de trou qui n'est pas occupée par le marqueur (26 ; 40), jusqu'aux deux orifices.

2. Endoprothèse selon la revendication 1, **caractérisée en ce que** l'une au moins des surfaces interne et externe de l'armature, au niveau d'un orifice du trou (20 ; 36), a une courbure, et le matériau adhésif (30, 32) est disposé avec une courbure correspondante.

3. Endoprothèse selon l'une des revendications 1 et 2, **caractérisée en ce que** son armature se prolonge par au moins un oeillet (18) relié à une des extrémités longitudinales de l'armature, et le trou (20 ; 36) est situé dans l'oeillet (18).

4. Endoprothèse selon l'une quelconque des revendications 1 à 3, **caractérisée en ce que** toute la surface du marqueur (26 ; 40) qui n'est pas en contact avec les parois du trou (20 ; 36) est recouverte de matériau adhésif.

5. Endoprothèse selon l'une quelconque des revendications 1 à 4, **caractérisée en ce que** le trou (20 ; 36) comporte une partie tronconique sur laquelle se trouvent les emplacements de contact avec le marqueur (26 ; 40).

6. Endoprothèse selon l'une quelconque des revendications 1 à 5, **caractérisée en ce que** le marqueur (26 ; 40) comporte une partie de forme cylindrique dont une circonférence comprend les emplacements de contact avec la paroi du trou (20 ; 36).

7. Endoprothèse selon l'une quelconque des revendications 1 à 6, **caractérisée en ce que** le matériau adhésif est choisi parmi les résines époxydes, acryliques, polyuréthannes et polyesters.

8. Procédé de pose d'un marqueur dans un trou débouchant d'une endoprothèse, le trou (20 ; 36) ayant deux orifices (22, 24) qui diffèrent au moins par la forme ou la dimension, le marqueur (26 ; 40) ayant au moins une forme ou une dimension telle que, avec son orientation dans le trou (20 ; 36), il peut passer entièrement par l'un des deux orifices (22) mais pas par l'autre (24), **caractérisé en ce qu'**il comprend :
l'introduction du marqueur (26 ; 40) dans le trou par l'orifice le plus grand (22) jusqu'à ce qu'il soit au contact de la paroi du trou (20 ; 36) avant l'autre orifice,
l'introduction de matériau adhésif fluide (30)d'un premier côté du marqueur (26 ; 40),
le durcissement du matériau adhésif,
l'introduction de matériau adhésif fluide (32) de l'autre côté du marqueur (26 ; 40), et
le durcissement du matériau adhésif.

9. Procédé de pose selon la revendication 8, **caractérisé en ce qu'**il comprend, pendant l'introduction de matériau adhésif fluide (30) du premier côté du marqueur (26 ; 40), l'obturation de l'orifice placé de l'autre côté du marqueur (26 ; 40).

10. Procédé de pose selon l'une des revendications 8 et 9, **caractérisé en ce qu'**un matériau adhésif est de type polymérisable, une étape de durcissement au moins de matériau adhésif est une polymérisation, et le procédé comprend une étape de recuit postérieure à la polymérisation.

## Claims

1. Endoprosthesis to be implanted in a duct of a living body, comprising:
a substantially tubular frame which is comprised between two longitudinal ends and which has an internal surface and an external surface,
the frame having at least one hole (20 ; 36) opening out via two orifices (22, 24) which differ at least in shape or size and which are located on the internal surface and on the external surface, respectively, of the tubular frame, and
a radio-opaque marker (26 ; 40) having at least a shape or size such that, with its orientation in the hole, it can pass completely through one of the two orifices (22) but not through the other (24), the marker being accommodated at least almost completely in the hole,
**characterised in that** the marker (26 ; 40) comprises an end portion in contact with at least several sites (28 ; 38) on the wall of the hole (20 ; 36) that are closer to one orifice (24) of the hole than to the other (22), and
an adhesive material (30, 32) fills the hole portion that is not occupied by the marker (26 ; 40), as far as the two orifices.

2. Endoprosthesis according to claim 1, **characterised in that** at least one of the internal and external surfaces of the frame, in the region of an orifice of the hole (20 ; 36), has a curvature, and the adhesive material (30, 32) is disposed with a corresponding curvature.

3. Endoprosthesis according to either of claims 1 and 2, **characterised in that** its frame continues with at least one eyelet (18) connected to one of the longitudinal ends of the frame, and the hole (20 ; 36) is located in the eyelet (18).

4. Endoprosthesis according to any one of claims 1 to 3, **characterised in that** all of the surface of the marker (26 ; 40) that is not in contact with the walls of the hole (20 ; 36) is covered with adhesive material.

5. Endoprosthesis according to any one of claims 1 to 4, **characterised in that** the hole (20 ; 36) comprises a frusto-conical portion on which the sites of contact with the marker (26 ; 40) are located.

6. Endoprosthesis according to any one of claims 1 to 5, **characterised in that** the marker (26 ; 40) comprises a cylindrical portion, a circumference of which comprises the sites of contact with the wall of the hole (20 ; 36).

7. Endoprosthesis according to any one of claims 1 to 6, **characterised in that** the adhesive material is selected from epoxy, acrylic, polyurethane and polyester resins.

8. Process for fitting a marker in a hole opening out from an endoprosthesis, the hole (20 ; 36) having two orifices (22, 24) which differ at least in shape or size, the marker (26 ; 40) having at least a shape or size such that, with its orientation in the hole (20 ; 36), it can pass completely through one of the two orifices (22) but not through the other (24), **characterised in that** it comprises:
the introduction of the marker (26 ; 40) into the hole through the larger orifice (22) until it is in contact with the wall of the hole (20, 36) upstream of the other orifice,
the introduction of fluid adhesive material (30) from a first side of the marker (26 ; 40),
the curing of the adhesive material,
the introduction of fluid adhesive material (32) from the other side of the marker (26 ; 40) and
the curing of the adhesive material.

9. Process according to claim 8, **characterised in that** it comprises, during the introduction of the fluid adhesive material (30) from the first side of the marker (26 ; 40), the closing off of the orifice located on the other side of the marker (26 ; 40).

10. Process according to either of claims 8 and 9, **characterised in that** an adhesive material is of the polymerisable type, one curing step at least in respect of the adhesive material is a polymerisation, and the process comprises a post-curing step subsequent to the polymerisation.

## Patentansprüche

1. Endoprothese zum Implementieren in einen Gang eines lebenden Körpers, mit:
einer im Wesentlichen röhrenförmigen Tragstruktur zwischen zwei Langsenden und mit einer internen Fläche und einer externen Fläche,
wobei die Tragstruktur mindesten ein Loch (20; 36) hat, das in zwei Öffnungen (22, 24) einmündet, die mindesten durch die Form oder die Größe sich voneinander unterscheiden, und die jeweils an der internen Fläche und der externe Fläche der röhrenförmigen Tragstruktur sich befinden, und
einem strahlenundurchlässigen Marker (26; 40) mindesten mit einer solchen Form oder Größe, dass er wegen seiner Ausrichtung im Loch völlig durch die eine Öffnung (22), aber nicht durch die andere (24), durchgehen kann, wobei der Marker mindesten fast völlig im Loch gelagert ist,
**dadurch gekennzeichnet, dass** der Marker (26; 40) einen Endteil in Kontakt mindesten mit mehreren Stellen (28; 38) der Wand des Lochs (20; 36) aufweist, die näher an einer Öffnung (24) des Lochs als die andere (22) sind, und dass
ein Klebestoff (30, 32) den Lochteil ohne Marker (26, 40) bis zu den beiden Öffnungen ausstopft.

2. Endoprothese nach Anspruch 1, **dadurch gekennzeichnet dass** mindesten eine der internen und externen Flächen der Tragstruktur im Bereich einer Öffnung des Lochs (20; 36) eine Krümmung aufweist, und der Klebestoff (30, 32) mit einer entsprechenden Krümmung angeordnet ist.

3. Endoprothese nach einem der Ansprüche 1 und 2, **dadurch gekennzeichnet dass** ihre Tragstruktur durch mindesten eine mit einer der Langsenden der Tragstruktur verbundene Öse (18) verlängert wird, und dass das Loch (20; 36) in der Öse liegt.

4. Endoprothese nach einem der Ansprüche 1 bis 3, **dadurch gekennzeichnet dass** die ganze Fläche des Markers (26; 40), die nicht die Wände des Lochs (20; 36) berührt, mit einer Klebestoffabdeckung versehen ist.

5. Endoprothese nach einem der Ansprüche 1 bis 4, **dadurch gekennzeichnet dass** das Loch (20, 36) einen kegelstumpfartigen Teil aufweist, an dem sich die Kontaktstellen mit dem Marker (26; 40) befinden.

6. Endoprothese nach einem der Ansprüche 1 bis 5, **dadurch gekennzeichnet dass** der Marker (26; 40) einen zylinderförmigen Teil aufweist, von dem ein Umfang die Kontaktstellen mit der Wand des Lochs (20; 36) aufweist.

7. Endoprothese nach einem der Ansprüche 1 bis 6, **dadurch gekennzeichnet dass** der Klebestoff unter den Epoxyd-, Acryl-, Polyurethan- und Polyesterharzen ausgewählt wird.

8. Einlegeverfahren für einen Marker in ein in eine Endoprothese einmündendes Loch, wobei das Loch (20, 36) zwei Öffnungen aufweist, die mindesten durch die Form oder die Größe sich voneinander unterscheiden, wobei der Marker (26; 40) mindesten eine solche Form oder Größe hat, dass er wegen seiner Ausrichtung im Loch völlig durch die eine Öffnung (22), aber nicht durch die andere (24), durchgehen kann, **dadurch gekennzeichnet dass** es umfasst:
die Einführung des Markers (26; 40) in das Loch durch die größte Öffnung (22), bis er die Wand des Lochs (20; 36) vor der anderen Öffnung berühre,
die Einführung eines flüssigen Klebestoffs (30) auf einer ersten Seite des Markers (26; 40),
die Aushärtung dieses Klebestoffs,
die Einführung eines flüssigen Klebestoffs (32) auf der anderen Seite des Markers (26; 40), und
die Aushärtung dieses Klebestoffs.

9. Einlegeverfahren nach Anspruch 8, **dadurch gekennzeichnet dass** es während der Einführung des flüssigen Klebestoffs (30) auf der ersten Seite des Markers (26; 40) den Verschluss der Öffnung auf der anderen Seite des Markers (26; 40) aufweist.

10. Einlegeverfahren nach einem der Ansprüche 8 und 9, **dadurch gekennzeichnet dass** ein Klebestoff ein polymerisierbarer Klebestoff ist, dass mindesten ein Aushärtungsschritt für den Klebestoff eine Polymerisierung ist, und dass das Verfahren einen Glühschritt nach der Polymerisierung aufweist.
